# NOUVEAU FASCICULE DE BREVET EUROPEEN

(11) **EP 0 973 486 B2**
(45) Date de publication et mention de la décision concernant l'opposition: **12.09.2007**
(45) Mention de la délivrance du brevet: 29.01.2003
(21) Numéro de dépôt: 98917296.0
(22) Date de dépôt: 02.04.1998
(51) Int. Cl.: A61Q 5/02, A61Q 5/12, A61K 8/81, A61K 8/891, A61K 8/895

(54) **COMPOSITIONS COSMETIQUES CONTENANT UN POLYMERE CATIONIQUE DE FAIBLE MASSE MOLECULAIRE ET UNE SILICONE ET LEURS UTILISATIONS**
KOSMETISCHE ZUSAMMENSETZUNGEN ENTHALTEND EIN KATIONISCHES POLYMER MIT NIEDRIGEM MOLEKULARGEWICHT UND EINEM SILIKON SOWIE DEREN VERWENDUNGEN
COSMETIC COMPOSITIONS CONTAINING A CATIONIC POLYMER WITH LOW MOLECULAR MASS AND A SILICON AND USES THEREOF

(30) Priorité: 07.04.1997 FR 9704221
(43) Date de publication de la demande: 26.01.2000
(73) Titulaire: L'ORÉAL, 75008 Paris (FR)
(72) Inventeur: DECOSTER, Sandrine, F-93800 Epinay sur Seine (FR); BEAUQUEY, Bernard, F-92110 Clichy (FR)
(74) Mandataire: Le Blainvaux Bellegarde, Françoise
(86) Numéro de dépôt international: PCT/FR1998/000670
(87) Numéro de publication internationale: WO 1998/044897

(56) Documents cités:
- EP-A- 0 059 428
- EP-A- 0 089 749
- EP-A- 0 115 252
- EP-A- 0 219 830
- EP-A- 0 521 748
- EP-A- 0 526 279
- WO-A-93/08787
- WO-A-94/03152
- WO-A-94/06403
- WO-A-98/14165
- DE-A- 3 029 306
- JP-A- 57 126 409

## Description

La présente invention concerne de nouvelles compositions cosmétiques comprenant dans un milieu cosmétiquement acceptable au moins une silicone et au moins un polymère de faible masse moléculaire comportant des unités cationiques.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes. En outre, sur des cheveux sensibilisés, il est connu d'utiliser de préférence un mélange de silicone et de polymère cationique.
Cependant, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibre affectant le coiffage. Ces inconvénients sont accentués dans le cas de cheveux fins, qui manquent de nervosité et de volume.
Le document EP526279 décrit une composition cosmétique capillaire comprenant une silicone hydroxyacylamino et un polymère substantif. Le document EP219830 décrit une composition comprenant une silicone carboxy et un polymère cationique quelconque. Le document EP59428 décrit une composition cosmétique capillaire comprenant un dérivé de kératine, un polymère cationique et un sel ou de l'urée et éventuellement une silicone. Ces compositions ne donnent pas encore satisfaction.

L'invention a donc pour but de proposer des compositions cosmétiques présentant des propriétés cosmétiques améliorées, en particulier au niveau du toucher des cheveux ou de la peau.

Or, la demanderesse a maintenant trouvé qu'un polymère cationique défini ci-après de masse moléculaire moyenne en poids comprise entre 8.000 et 17.000 permettait d'atteindre ces buts.
L'invention a ainsi pour objet une composition cosmétique, caractérisée en ce qu'elle comprend, dans un milieu cosmétiquement acceptable, au moins une silicone et au moins un polymère ou un copolymère comprenant au moins des motifs de formules (I) et/ou (II) définies ci-après de masse moléculaire moyenne en poids comprise entre 8.000 et 17.000.

Un autre objet de l'invention concerne un procédé de traitement des matières kératiniques, telles que les cheveux, caractérisé en ce qu'il consiste à appliquer sur lesdites matières des compositions cosmétiques selon l'invention.

Les différents objets de l'invention vont maintenant être détaillés. L'ensemble des significations et définitions des composés utilisés dans la présente invention données ci-dessous sont valables pour l'ensemble des objets de l'invention.

Au sens de la présente invention, l'expression "polymère cationique" désigne tout polymère contenant au moins des groupements cationiques et/ou des groupements ionisables en groupements cationiques. Ainsi des copolymères comportant également des groupements anioniques seront désignés par le terme "polymère cationique".

Les compositions conformes à l'invention comprennent nécessairement un polymère ou un copolymère cationique comportant au moins des motifs répondant aux formules (I) et/ou (II) : 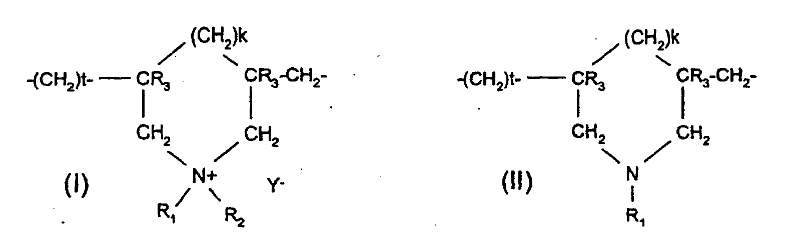 formules dans lesquelles k et t sont égaux à 0 ou 1, la somme k + t étant égale à 1 ; R₃ désigne un atome d'hydrogène ou un radical méthyle ; R₁ et R₂, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (1 à 5 atomes de carbone) ou R₁ et R₂ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.
Ces polymères peuvent être notamment préparés selon les brevets US 3.996.146 et US 3.288.770.

De préférence , R₁ et R₂ désignent indépendamment l'un de l'autre, méthyle ou éthyle et R₃ désigne un atome d'hydrogène.

Les polymères cationiques utilisés ont de préférence une masse moléculaire moyenne en poids comprise entre 8000 et 15.000 environ.

Les masses moléculaires moyennes en poids sont mesurées par GPC (Chromatographie par perméation de gel).

La présente invention concerne également les copolymères comprenant des motifs de formule (I) et/ou (II). Les copolymères comprennent des motifs résultant de la copolymérisation avec les acides acrylique ou méthacrylique, les méthacrylates d'alkyle en C₁-C₁₂, les acrylates d'alkyle en C₁-C₁₂, l'acrylamide.

Parmi les polymères définis ci-dessus, on peut citer plus particulièrement l'homopolymère de chlorure de diallyldiméthylammonium de masse moléculaire moyenne en poids comprise entre 9500 et 12500.

Selon l'invention, le ou les polymère cationique de faible masse moléculaire peuvent représenter de 0,001 % à 10 % en poids, de préférence de 0,005 % à 5 % en poids, et encore plus préférentiellement de 0,01 % à 3 % en poids, du poids total de la composition finale.

Les silicones utilisables conformément à l'invention peuvent être solubles ou insolubles dans l'eau ou dans la composition finale. Elles peuvent être volatiles ou non volatiles.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 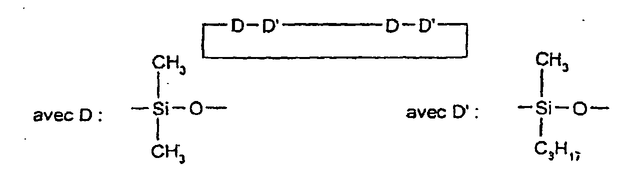 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics". On utilise de préférence des silicones non volatiles et plus particulièrement (i) les polyalkylsiloxanes ;
   (ii) les polyarylsiloxanes ;
   (iii) les polyalkylarylsiloxanes;
   (iv) les gommes de silicone ;
   (v) les résines de silicone ;
   (vi) les polyorganosiloxanes, comportant dans leur structure générale, un ou plusieurs groupements organofonctionnels directement fixés sur la chaîne siloxanique ou fixés par l'intermédiaire d'un radical hydrocarboné ;
   (vii) les copolymères blocs ayant un bloc linéaire polysiloxane-polyoxyalkylène comme unités répétitives ;
   (viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
   (ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
   (x) ou leurs mélanges.

Parmi les polyalkylsiloxanes, on peut citer principalement :
- les polydiméthylsiloxanes linéaires à groupements terminaux triméthylsilyle, comme par exemple, et à titre non limitatif, les huiles SILBIONE de la série 70047 commercialisées par RHONE POULENC, l'huile 47 V 500 000 de RHONE POULENC ou certaines VISCASIL de la GENERAL ELECTRIC ;
- les polydiméthylsiloxanes linéaires à groupements terminaux hydroxydiméthylsilyle telles que les huiles de la série 48 V de RHONE POULENC.

Dans cette classe de polyalkylsiloxanes, on peut également mentionner les polyalkylsiloxanes vendus par la société GOLDSCHMIDT sous les dénominations commerciales ABILWAX 9800 et ABILWAX 9801 qui sont des polyalkyl(C₁-C₂₀)siloxanes.

Parmi les polyalkylarylsiloxanes, on peut citer les polydiméthylméthylphénylsiloxanes ou les polydiméthyldiphénylsiloxanes, linéaires ou ramifiés tels que le produit DC 556 COSMETIC GRAD FLUID de DOW CORNING.

Les gommes de silicone, conformes à l'invention, sont des polyorganosiloxanes de masse moléculaire moyenne en nombre comprise entre 200.000 et 1.000.000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On cite, par exemple, les composés suivants :
- polydiméthylsiloxane,
- poly[(diméthylsiloxane)/(méthylvinylsiloxane)],
- poly[(diméthylsiloxane)/(diphénylsiloxane)].
- poly((diméthylsiloxane)/(phénylméthylsiloxane)],
- poly[(diméthylsiloxane)/(diphénylsiloxane)/(méthylvinylsiloxane)],

On peut citer, par exemple, les mélanges suivants :
**1)** les mélanges formés à partir d'un polydiméthylsiloxane hydroxylé en bout de chaîne (DIMETHICONOL selon la nomenclature CTFA) et d'un polydiméthylsiloxane cyclique (CYCLOMETHICONE selon la nomenclature CTFA) tels que le produit Q2 1401 vendu par la société DOW CORNING ;
**2)** les mélanges formés à partir d'une gomme de polydiméthylsiloxane avec une silicone cyclique tels que le produit SF 1214 SILICONE FLUID de GENERAL ELECTRIC qui est une gomme SE 30 de poids moléculaire 500.000 solubilisée dans la SF 1202 SILICONE FLUID (décaméthylcyclopentasiloxane) ;
**3)** les mélanges de deux polydiméthylsiloxanes (PDMS) de viscosité différente, notamment d'une gomme PDMS et d'une huile PDMS tels que les produits SF 1236 et CF 1241 de la GENERAL ELECTRIC. Le produit SF 1236 est le mélange d'une huile SE 30 définie ci-dessus de viscosité 20 m²/s et d'une huile SF 96 de viscosité 5.10⁻⁵ m²/s (15% de gomme SE 30 et 85% d'huile SF 96). Le produit CF 1241 est le mélange d'une gomme SE 30 (33%) et d'une PDMS (67%) de viscosité 10⁻³ m²/s.

Les résines de silicone conformes à l'invention sont de préférence des systèmes siloxaniques réticulés renfermant les unités :
R₂SiO_{2/2}, RSiO_{3/2}, SiO_{4/2}, dans lesquelles R désigne un groupe hydrocarboné possédant 1 à 6 atomes de carbone ou un groupement phényle.Parmi ces produits, ceux particulièrement préféres sont ceux où R désigne un radical alkyle inférieur ou phényle.

Parmi ces résines, on peut citer le produit vendu sous le nom DOW CORNING 593 par DOW CORNING ou ceux vendus sous le nom SILICONE FLUID SS 4267 par la GENERAL ELECTRIC et qui sont des diméthyl/triméthypolylsiloxanes.

Les polyorganosiloxanes organomodifiés de l'invention sont des polysiloxanes tels que définis précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les polysiloxanes comportant :
a) des groupements polyéthylèneoxy et/ou polypropylèneoxy, comportant éventuellement des groupes alkyle, tels que le produit dénommé lauryl méthicone copolyol vendu sous le nom Q2 5200 par DOW CORNING ;
b) des groupements (per)fluorés, comme les groupements trifluoroalkyls tels que, par exemple, ceux vendus par SHIN ETSU sous le nom FL 100 ;
c) des groupements thiols ;
d) des groupements carboxylates, tels que les produits décrits dans le brevet européen EP 185 507 de CHISSO CORPORATION ;
e) des groupements hydroxylés, tels que les polyorganopolysiloxanes à fonction hydroxyalkyle décrits dans la demande de brevet français FR 85-16334 et en particulier les polyorganopolysiloxanes à fonction y- hydroxypropyle ;
f) des groupements alcoxylés comportant au moins 12 atomes de carbone, tels que le produit SILICONE COPOLYMER F755 de SWS SILICONES et les produits ABILWAX 2428, ABILWAX 2434, ABILWAX 2440 de la société GOLDSCHMIDT.
g) des groupements acyloxyalkyls comportant au moins 12 atomes de carbone, tels que les polyorganosiloxanes décrits dans la demande de brevet français FR 88-17433 et en particulier les polyorganosiloxanes à fonction stéaroyloxypropyle.
h) des groupements amphotères;
i) des groupements bisulfites.
j) des groupements hydroxyacylamino, comme les polyorganosiloxanes décrits dans la demande EP 342 834. On peut citer par exemple le produit Q2-8413 de la société DOW CORNING.
k) des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ; On utilise plus particulièrement les silicones dénommées amodiméthicone et triméthylsilylamodiméthicone selon la dénomination CTFA ;

Les copolymères blocs ayant un bloc linéaire polysiloxane-polyoxyalkylène comme unités répétitives utilisés dans le cadre de la présente invention ont de préférence la formule générale suivante :

([ Y (R₂SiO)ₐ R'₂SiYO] [(CnH₂ₙO)_{b}])_{c} (V)

dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5, de préférence compris entre 5 et 200 et encore plus particulièrement entre 5 et 100.
- b est un nombre entier supérieur ou égal à 4, de préférence compris entre 4 et 200 et encore plus particulièrement entre 5 et 100.
- c est un nombre entier supérieur ou égal à 4, de préférence compris entre 4 et 1000 et encore plus particulièrement entre 5 et 300.

- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10 % environ à 95 % environ en poids du copolymère bloc,
- le poids moléculaire moyen en poids du copolymère bloc étant d'au moins 3.000 et de préférence compris entre 5000 et 1000000 et encore plus particulièrement entre 10000 et 200000.

R et R' sont préférentiellement choisis parmi le groupe comprenant les radicaux alkyls comme par exemple les radicaux méthyle, éthyle, propyle, butyle, pentyle, hexyle, octyle, décyle, dodécyle, les radicaux aryls comme par exemple phényle, naphtyle, les radicaux aralkyls comme par exemple benzyle, phényléthyle, les radicaux tolyle, xylyle et cyclohexyle.

Y est de préférence -R"-, -R"-CO-, -R"-NHCO-, -R"-NH-CO-NH-R"'-NHCO, -R"-OCONH-R"'-NHCO-, où R" est un groupe alkylène divalent comme par exemple l'éthylène, le propylène ou le butylène et R"' est un groupe alkylène divalent ou un groupe arylène divalent comme -C₆H₄-, -C₆H₄-C₆H₄-, -C₆H₄-CH₂-C₆H₄-, -C₆H₄-C(CH₃)₂-C₆H₄-.

Encore plus préférentiellement, Y représente un radical alkylène divalent, plus particulièrement le radical -CH₂-CH₂-CH₂- ou le radical C₄H₈.

La préparation des copolymères blocs mis en oeuvre dans le cadre de la présente invention est décrite dans la demande européenne EP 0 492 657 A1, dont l'enseignement est inclus à titre de référence dans la présente description.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0 412 704, EP-A-0 412 707, EP-A-0 640 105 et WO 95/00578 dont les enseignements sont totalement inclus dans la présente description à titre de références non limitatives. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Des exemples de polymères à squelette polysiloxanique greffé par des monomères organiques non siliconés convenant à la mise en oeuvre de la présente invention, ainsi que leur mode particulier de préparation, sont notamment décrits dans les demandes de brevets EP-A- 0 582 152, WO 93/23009 et WO 95/03776 dont les enseignements sont totalement inclus dans la présente description à titre de références non limitatives.

Selon l'invention, toutes les silicones peuvent également être utilisées sous forme d'émulsions ou de microémulsions.

Les silicones particulièrement préférées conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;
- les mélanges d'organopolysiloxanes et de silicones cycliques tels que le produit Q2 1401 commercialisé par la société DOW CORNING, et le produit SF 1214 commercialisé par la société GENERAL ELECTRIC ;
- les mélanges de deux PDMS de viscosités différentes notamment d'une gomme et d'une huile tels que le produit SF 1236 commercialisé par la société GENERAL ELECTRIC ;
- la résine d'organopolysiloxane commercialisée sous la dénomination DOW CORNING 593 ;
- les polysiloxane à groupements aminés tels que les amodiméthicone ou les triméthylsilylamodiméthicone ;

Selon l'invention, la ou les silicone peuvent représenter de 0,001 % à 10 % en poids, de préférence de 0,005 % à 5 % en poids, et encore plus préférentiellement de 0,01 % à 3 % en poids, du poids total de la composition finale.

Les compositions de l'invention contiennent en outre avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides olêique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

On peut également utiliser des tensioactifs cationiques parmi lesquels on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

La composition de l'invention peut également contenir au moins un additif choisi parmi les épaississants, les tensioactifs, les parfums, les agents nacrants, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 50% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques et cationiques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 2 % à 50 % en poids, de préférence de 10 % à 35 % en poids, et encore plus préférentiellement de 12 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être également des lotions de mise en plis, des lotions pour le brushing, des compositions de fixation (laques) et de coiffage. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également de compositions de maquillage telles que des fonds de teint, des crèmes de jour teintées, des mascaras, des fards à joues et à paupières, des rouges à lèvres, des vernis à ongles.
Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1

On a réalisé deux compositions de shampooing, l'une conforme à l'invention (composition A) et l'autre comparative (composition B) :

| | A Invention | B Comparatif |
|---|---|---|
| - Lauryléthersulfate de sodium (C₁₂/C₁₄ à 70/30) à 2,2 moles d'oxyde d'éthylène (MA = matière active) | 15,5 gMA | 15,5 gMA |
| - Disodium cocoampho diacétate (*) | 2,56 gMA | 2,56 gMA |
| - Silicone aminée (***) | 1,05 gMA | 1,05 gMA |
| - Silicone (60 000 cSt) (****) | 2 g | 2 g |
| - Homopolymère de chlorure de diallyl diméthylammonium de Mw ≈ 11000 | 0,6 g | ― |
| - Homopolymère de chlorure de diallyl diméthylammonium de Mw ≈ 400.000 (MERQUAT 100 de CALGON) | ― | 0,6 g |
| - Distéarate d'éthylèneglycol | 1,5 g | 1,5 g |
| - Acide polyacrylique réticulé (**) | 0,2 g | 0,2 g |
| - Monoisopropanolamide d'acides de coprah | 0,9 g | 0,9 g |
| - Acide citrique qs pH | 5 | 5 |
| - Eau déminéralisée qs | 100 g | 100 g |

| | | |
|---|---|---|
| (*) MIRANOL C2M commercialisé par RHONE POULENC (**) CARBOPOL 980 commercialisé par GOODRICH (***) : Amodiméthicone commercialisée en émulsion cationique à 35% de matière active sous la dénomination FLUID DC 939 par la société DOW CORNING (****) : Polydiméthylsiloxane de viscosité 60 000 cSt commercialisé par la société DOW CORNING sous la dénomination FLUID DC 200- 60 000 cSt. | | |

On effectue un shampooing en appliquant environ 12 g de la composition A sur des cheveux naturels préalablement mouillés. On fait mousser le shampooing puis on rince abondamment à l'eau.
On procède selon le même mode opératoire que ci-dessus avec la composition comparative B.

Un panel d'experts évalue le démêlage, le toucher des cheveux mouillés, le démêlage, le toucher, la légèreté, la douceur et le lissage des cheveux séchés.

Le toucher des cheveux traités avec la composition A est naturel et sans charge alors que ceux traités avec la composition B contenant un polymère de même structure mais de masse moléculaire élevée ont un toucher chargé. Les cheveux ne semblent pas propres.

### EXEMPLE 2

On a préparé un shampooing de composition suivante :
- Lauryléthersulfate de sodium (C₁₂/C₁₄ à 70/30) à 2,2 moles d'oxyde d'éthylène en solution aqueuse à 70% de MA (MA = matière active) 15,5 gMA
- Disodium cocoampho diacétatet(*) 2,56gMA
- Polydiméthylsiloxane en émulsion aqueuse à 50%MA (***) 2,7 g
- Homopolymère de chlorure de diallyl diméthylammonium de Mw ≈ 11000 0,6 g
- Distéarate d'éthylèneglycol 1,5 g
- Monoisopropanolamide d'acides de coprah 0,9 g
- Acide polyacrylique réticulé (**) 0,2 g
- Acide citrique qs pH 5
- Parfum, Conservateurs qs
- Eau déminéralisée qs 100 g
(*) MIRANOL C2M commercialisé par RHONE POULENC
(***) : Polydiméthylsiloxane (60.000 Cst.) en émulsion non ionique à 50% de matière active commercialisée sous la dénomination DC 2-1691 par la société DOW CORNING
(**) CARBOPOL 980 commercialisé par GOODRICH

### EXEMPLE 3

On a préparé un shampooing de composition suivante :
- Lauryléthersulfate de sodium (C₁₂/C₁₄ à 70/30) à 2,2 moles d'oxyde d'éthylène en solution aqueuse à 70% de MA (MA = matière active) 15,5 gMA
- Cocobétaïne à 32% MA(*) 3,2 gMA
- Silicone aminée (***) 1,05 g
- Silicone (60 000 cSt) (****) 2 g
- Homopolymère de chlorure de diallyl diméthyl ammonium de Mw ≈ 11000 0,6 g
- Hydrolysat de protéine de blé quaternisée 0,1 gMA
- Mono et distéarate d'éthylèneglycol 1,5 g
- Monoisopropanolamide d'acides de coprah 1 g
- Acide polyacrylique réticulé (**) 0,2 g
- Acide citrique qs pH 5
- Parfum, Conservateurs qs
- Eau déminéralisée qs 100 g
(*) DEHYTON AB 30 de HENKEL
(***) : Amodiméthicone commercialisée en émulsion cationique à 35% de matière active sous la dénomination FLUID DC 939 par la société DOW CORNING
(****) : Polydiméthylsiloxane de viscosité 60 000 cSt commercialisé par la société DOW CORNING sous la dénomination FLUID DC 200 - 60 000 cSt.

## Revendications

1. Composition cosmétique, **caractérisée par le fait qu'**elle comprend, dans un milieu cosmétiquement acceptable, au moins une silicone et au moins un polymère cationique choisi parmi les polymères ou les copolymères comportant au moins des motifs répondant aux formules (I) et/ou (II) : 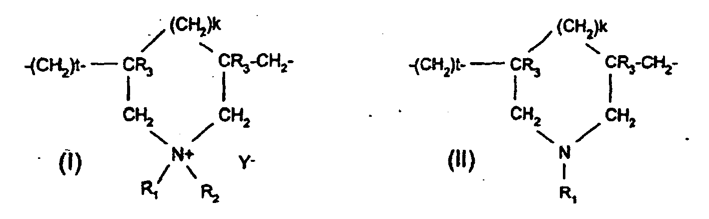 formules dans lesquelles k et t sont égaux à 0 ou 1, la somme k + t étant égale à 1 ; R₃ désigne un atome d'hydrogène ou un radical méthyle ; R₁ et R₂, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (1 à 5 atomes de carbone) ou R₁ et R₂ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate, la masse moléculaire moyenne en poids du polymère étant comprise entre 8 000 et 17 000.

2. Composition selon la revendication précédente, **caractérisée par le fait que** R₁ et R₂, indépendamment l'un de l'autre, désignent méthyle ou éthyle et R₃ désigne un atome d'hydrogène.

3. Composition selon l'une quelconque des revendications précédentes, **caractérisée par le fait que** ledit polymère cationique est choisi parmi les homopolymères du chlorure de diméthyldiallylammonium.

4. Composition selon l'une quelconque des revendications précédentes, **caractérisée par le fait que** la masse moléculaire moyenne en poids est comprise entre 8000 et 15000.

5. Composition selon l'une quelconque des revendications précédentes, **caractérisée par le fait que** les silicones sont choisies dans le groupe constitué par :
(i) les polyalkylsiloxanes ;
(ii) les polyarylsiloxanes ;
(iii) les polyalkylarylsiloxanes ;
(iv) les gommes de silicone ;
(v) les résines de silicone ;
(vi) les polyorganosiloxanes, comportant dans leur structure générale, un ou plusieurs groupements organofonctionnels directement fixés sur la chaîne siloxanique ou fixés par l'intermédiaire d'un radical hydrocarboné ;
(vii) les copolymères blocs ayant un bloc linéaire polysiloxane-polyoxyalkylène comme unités répétitives ;
(viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(x) ou leurs mélanges.

6. Composition selon l'une quelconque des revendications précédentes, **caractérisée par le fait qu'**elle comprend en outre au moins un agent tensioactif choisi parmi les tensioactifs anioniques, cationiques, non ioniques, amphotères et leurs mélanges.

7. Compositions selon la revendication 6, **caractérisées par le fait que** le ou les agents tensioactifs sont présents à une concentration comprise entre 0,1% et 60% en poids, de préférence entre 3% et 40% en poids, et encore plus préférentiellement entre 5% et 30% en poids, par rapport au poids total de la composition.

8. Composition selon l'une quelconque des revendications précédentes, **caractérisée en ce que** le polymère cationique est présent à une concentration comprise entre 0,001 % et 10 % en poids par rapport au poids total de la composition, de préférence entre 0,005 % et 5 % en poids et en particulier entre 0,01 % et 3 % en poids.

9. Composition selon l'une quelconque des revendications précédentes, **caractérisée en ce que** la silicone est présente à une concentration comprise entre 0,001 % et 10 % en poids par rapport au poids total de la composition, de préférence entre 0,005 % et 5 % en poids et en particulier entre 0,01 % et 3 % en poids.

10. Composition selon l'une quelconque des revendications précédentes, **caractérisées par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

11. Utilisation d'une composition telle que définie dans l'une quelconque des revendications précédentes pour le lavage des matières kératiniques.

12. Procédé de traitement des matières kératiniques, telles que les cheveux, **caractérisé en ce qu'**il consiste à appliquer sur lesdites matières une composition cosmétique selon l'une des revendications 1 à 10.

## Claims

1. Cosmetic composition, **characterized in that** it comprises, in a cosmetically acceptable medium, at least one silicone and at least one cationic polymer chosen from polymers or copolymers containing at least some units corresponding to formulae (I) and/or (II): 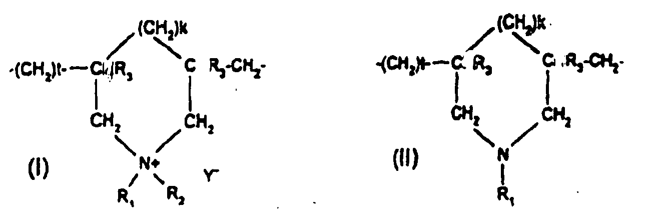 in which formulae k and t are equal to 0 or 1, the sum k + t being equal to 1; R₃ denotes a hydrogen atom or a methyl radical; R₁and R₂, independently of each other, denote an alkyl group having from 1 to 22 carbon atoms, a hydroxyalkyl group in which the alkyl group preferably has 1 to 5 carbon atoms, a lower amidoalkyl group (1 to 5 carbon atoms) or R₁ and R₂ can denote, together with the nitrogen atom to which they are attached, heterocyclic groups such as piperidyl or morpholinyl; Y⁻ is an anion such as bromide, chloride, acetate, borate, citrate, tartrate, bisulphate, bisulphite, sulphate or phosphate, the weight-average molecular mass of the polymer is between 8 000 and 17 000.

2. Composition according to the preceding claim, **characterized in that** R₁ and R₂, independently of each other, denote methyl or ethyl and R₃ denotes a hydrogen atom.

3. Composition according to either of the preceding claims, **characterized in that** the said cationic polymer is chosen from dimethyldiallylammonium chloride homopolymers.

4. Composition according to any one of the preceding claims, **characterized in that** the weight-average molecular mass is between 8000 and 15,000.

5. Composition according to any one of the preceding claims, **characterized in that** the silicones are chosen from the group consisting of:
(i) polyalkylsiloxanes;
(ii) polyarylsiloxanes;
(iii) polyalkylarylsiloxanes;
(iv) silicone gums;
(v) silicone resins;
(vi) polyorganosiloxanes containing in their general structure one or more organofunctional groups directly attached to the siloxane chain or attached via a hydrocarbon radical;
(vii) block copolymers having a linear polysiloxane-polyoxyalkylene block as repeating units;
(viii) grafted silicone polymers, with a non-silicone organic skeleton, consisting of an organic main chain formed from organic monomers containing no silicone, on which is grafted, inside the said chain as well as, optionally, on at least one of its ends, at least one polysiloxane macromonomer;
(ix) grafted silicone polymers, with a polysiloxane skeleton grafted with non-silicone organic monomers, comprising a polysiloxane main chain on which is grafted, inside the said chain as well as, optionally, on at least one of its ends, at least one organic macromonomer containing no silicone;
(x) or mixtures thereof.

6. Composition according to any one of the preceding claims, **characterized in that** it also comprises at least one surfactant chosen from anionic, cationic, nonionic and amphoteric surfactants, and mixtures thereof.

7. Compositions according to Claim 6, **characterized in that** the surfactant(s) is(are) present at a concentration of between 0.1% and 60% by weight, preferably between 3% and 40% by weight and even more preferably between 5% and 30% by weight, relative to the total weight of the composition.

8. Composition according to any one of the preceding claims, **characterized in that** the cationic polymer is present at a concentration of between 0.001% and 10% by weight relative to the total weight of the composition, preferably between 0.005% and 5% by weight and in particular between 0.01% and 3% by weight.

9. Composition according to any one of the preceding claims, **characterized in that** the silicone is present at a concentration of between 0.001% and 10% by weight relative to the total weight of the composition, preferably between 0.005% and 5% by weight and in particular between 0.01% and 3% by weight.

10. Composition according to any one of the preceding claims, **characterized in that** it is in the form of a shampoo, a conditioner, a composition for permanent-waving, straightening, dyeing or bleaching the hair, a rinse-out composition to be applied between the two steps of a permanent-waving or straightening operation on the hair, or a washing composition for the body.

11. Use of a composition as defined in any one of the preceding claims, for washing keratin substances.

12. Process for treating keratin substances, such as the hair, **characterized in that** it consists in applying a cosmetic composition according to one of Claims 1 to 10 to the said substances.

## Patentansprüche

1. Kosmetische Zusammensetzung, **dadurch gekennzeichnet, daß** sie in einem kosmetisch akzeptablen Medium mindestens ein Silicon und mindestens ein kationisches Polymer enthält, das unter den Polymeren und Copolymeren ausgewählt ist, die zumindest Einheiten der Formeln (I) und/oder (II) enthalten: 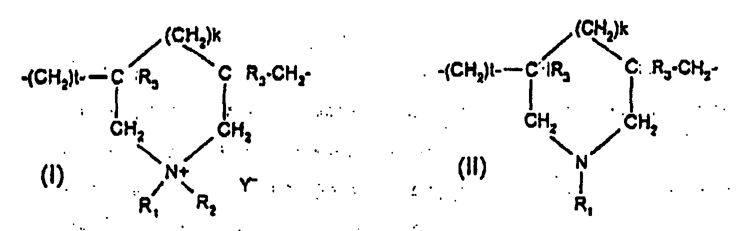 wobei in den Formeln:
k und t Null oder 1 bedeuten, wobei die Summe k + t 1 ist; R₃ ein Wasserstoffatom oder eine Methylgruppe bedeutet; R₁ und R₂ unabhängig voneinander eine Alkylgruppe mit 1 bis 22 Kohlenstoffatomen, eine Hydroxyalkylgruppe, worin die Alkylgruppe vorzugsweise 1 bis 5 Kohlenstoffatome aufweist, oder eine niedere (1 bis 5 Kohlenstoffatome) Amidoalkylgruppe bedeuten oder R₁ und R₂ zusammen mit dem Stickstoffatom, an das sie gebunden sind, heterocyclische Gruppen, wie Piperidinyl oder Morpholinyl, bilden können; und Y ein Anion ist, wie beispielsweise Bromid, Chlorid, Acetat, Borat, Citrat, Tartrat, Hydrogensulfat, Hydrogensulfit, Sulfat und Phosphat, und wobei das Gewichtsmittel der Molmasse des Polymers im Bereich von 8 000 bis 17 000 liegt.

2. Zusammensetzung nach dem vorhergehenden Anspruch, **dadurch gekennzeichnet, daß** die Gruppen R₁ und R₂ unabhängig voneinander Methyl oder Ethyl bedeuten und R₃ ein Wasserstoffatom ist.

3. Zusammensetzung nach einem der vorhergehenden Ansprüche, **dadurch gekennzeichnet, daß** das kationische Polymer unter den Homopolymeren von Dimethyldiallylammoniumchlorid ausgewählt ist.

4. Zusammensetzung nach einem der vorhergehenden Ansprüche, **dadurch gekennzeichnet, daß** das Gewichtsmittel der Molmasse im Bereich von 8 000 bis 15 000 liegt.

5. Zusammensetzung nach einem der vorhergehenden Ansprüche, **dadurch gekennzeichnet, daß** die Silicone ausgewählt sind unter:
(i) Polyalkylsiloxanen ;
(ii) Polyarylsiloxanen;
(iii) Polyalkylarylsiloxanen;
(iv) Silicongummis;
(v) Siliconharzen;
(vi) Polyorganosiloxanen, die in ihrer allgemeinen Struktur eine oder mehrere organofunktionelle Gruppen aufweisen, die direkt oder über eine Kohlenwasserstoffgruppe an die Siloxankette gebunden sind;
(vii) Blockcopolymeren, die als wiederkehrende Einheit einen geradkettigen Polysiloxan-Polyoxyalkylen-Block enthalten;
(viii) gepfropften Siliconpolymeren mit einem nicht siliconhaltigen organischen Grundgerüst, die aus einer organischen Hauptkette bestehen, die aus organischen Monomeren gebildet wird, die kein Silicon enthalten, auf die im Inneren der Kette sowie gegebenenfalls an mindestens einem ihrer Enden mindestens ein Polysiloxanmakromer gepfropft ist;
(ix) gepfropften Siliconpolymeren mit Polysiloxangerüst, auf das nicht siliconhaltige organische Monomere gepfropft sind, die eine Polysiloxanhauptkette enthalten, auf die im Inneren der Kette sowie gegebenenfalls an mindestens einem Kettenende mindestens ein organisches Monomer gepfropft ist, das kein Silicon enthält;
(x) oder deren Gemischen.

6. Zusammensetzung nach einem der vorhergehenden Ansprüche, **dadurch gekennzeichnet, daß** sie ferner mindestens einen grenzflächenaktiven Stoff enthält, der unter den anionischen, kationischen, nichtionischen oder amphoteren grenzflächenaktiven Stoffen oder deren Gemischen ausgewählt ist.

7. Zusammensetzung nach Anspruch 6, **dadurch gekennzeichnet, daß** der (die) grenzflächenaktive(n) Stoff(e) in einer Konzentration im Bereich von 0,1 bis 60 Gew.-%, vorzugsweise 3 bis 40 Gew.-% und noch bevorzugter 5 bis 30 Gew.-%, bezogen auf das Gesamtgewicht der Zusammensetzung, vorliegen.

8. Zusammensetzung nach einem der vorhergehenden Ansprüche, **dadurch gekennzeichnet, daß** das kationische Polymer in einer Konzentration im Bereich von 0,001 bis 10 Gew.-%, bezogen auf das Gesamtgewicht der Zusammensetzung, vorzugsweise 0,005 bis 5 Gew.-% und insbesondere 0,01 bis 3 Gew.-% vorliegt.

9. Zusammensetzung nach einem der vorhergehenden Ansprüche, **dadurch gekennzeichnet, daß** das Silicon in einer Konzentration im Bereich von 0,001 bis 10 Gew.-%, bezogen auf das Gesamtgewicht der Zusammensetzung, vorzugsweise 0,005 bis 5 Gew.-% und insbesondere 0,01 bis 3 Gew.-% vorliegt.

10. Zusammensetzung nach einem der vorhergehenden Ansprüche, **dadurch gekennzeichnet, daß** sie als Haarwaschmittel, Haarpflegespülung, Zusammensetzung für permanente Verformungen, zur Entkräuselung, zum Färben und zum Entfärben der Haare, Zusammensetzung, die ausgespült wird und zwischen den beiden Schritten einer Dauerwelle oder Entkräuselung aufgetragen wird, und reinigende Zusammensetzung für den Körper vorliegt.

11. Verwendung einer Zusammensetzung nach einem der vorhergehenden Ansprüche zum Waschen von Keratinsubstanzen.

12. Verfahren zur Behandlung von Keratinsubstanzen, wie dem Haar, **dadurch gekennzeichnet, daß** es darin besteht, auf die Keratinsubstanzen eine kosmetische Zusammensetzung nach einem der Ansprüche 1 bis 10 aufzutragen.
